# EUROPEAN PATENT APPLICATION

(11) **EP 1 302 114 A2**
(43) Date of publication of application: **16.04.2003**
(21) Application number: 02078931.9
(22) Date of filing: 23.09.2002
(51) Int. Cl.: A23L 1/30

(54) **Mixtures for stimulating glucose up-take**

(30) Priority: 16.10.2001 EP 01308783
(71) Applicant: UNILEVER N.V., 3013 AL Rotterdam (NL); UNILEVER PLC, London EC4P 4BQ (GB)
(72) Inventor: Brown, Anna Louise, Sharnbrook, Bedford MK44 1LQ (GB); Cain, Frederick William, c/o Loders Croklaan BV, 1521 AZ Wormerveer (NL); Mohede, Ingrid Celestina Maria, Loders Croklaan BV, 1521 AZ Wormerveer (NL); Parmar, Preyseh, c/o Unilver R&D Colworth, Bedford, MK44 1LQ (GB); Rogers, Julia Sarah, Sharnbrook, Bedford MK44 1LQ (GB); Schmid, Ulrike, c/o Loders Croklaan BV, 1521 AZ Wormerveer (NL)
(74) Representative: Sikken, Antonius H. J. M.

(57) **Abstract**

Our invention concerns a mixture of CLA glycerides, and / or CLA-fatty acids and or CLA-alkyl esters and another component, wherein the other component (= component A) is selected for its capacity to alleviate problems related to insulin resistance in mammals , using CLA rich diets and / or foods and / or food supplements using an appropriate in vitro test so that in at least one step of the in vitro test, as described in the text, an improvement in test results is obtained of at least 4 % by the blend of the CLA derivative and component A when compared to the CLA derivative only.

The inventon further concerns CLA rich dietic food, food supplements and foods containing the combination of CLA and component A as defined, while also the use of this combination for achieving an alleviation of insulin resistance is part of the invention.

## Description

Conjugated linoleic acid (= CLA ) and derivatives such as glycerides or alkyl esters or inorganic salts thereof are well known for a number of health effects. According to WO 99/29317 CLA can be used to treat diabetes by administering an effective amount of CLA to a human suffering from diabetes. The administration of CLA results in a modulation of the level of expression of certain genes involved in the regulation of lipid and glucose metabolism enzymes and / or in the regulation of adipocyte differentiation. It is concluded in this document that the administration of CLA can normalize glucose tolerance and reduce plasma insulin , triglyceride and free fatty acid levels. The CLA can be administered in any suitable form including powders or as mixtures with oils. The CLA also may contain antioxidants or free fatty acids.

We found that although CLA might affect the expression of genes as disclosed in WO 317 it can also reduce insulin stimulated glucose up-take by adipocytes from mammals. A reduction in insulin stimulated glucose uptake is a characteristic of insulin resistance. This reduction in insulin stimulated glucose up-take is an important property to address , because this property plays an essential role in the way the persons consuming the CLA may react to the intake of CLA. Therefore we tried in our study to find ways that could be used to stimulate the glucose up-take by adipocytes from mammals. This study resulted in the finding of novel mixtures that can be used for this purpose.
Therefore our invention concerns in the first instance a mixture of CLA glycerides, and/or CLA-fatty acids and or CLA-alkyl esters and / or salts and another component, wherein the other component (= component A) is selected for its capacity to stimulate glucose up-take in adipocytes from mammals, using an appropriate *in vitro* glucose up-take assay wherein the glucose up-take stimulation is at least 4 %, preferably at least 20% better by use of the blend of the CLA derivative and component A when compared to the use of the CLA or CLA derivative only.

### TEST PROTOCOL

### 1. Evaluation of glucose uptake in adipocytes

### Differentiation of adipocytes

Murine (3T3-L1 cells; ATCC CL-173) were seeded into multi-well plates and incubated in growth medium (DMEM supplemented with 10% FCS, 2mM L-glutamine, 100iu/ml penicillin and 100µg/ml streptomycin) at 37°C/5% CO2 until 2 days post-confluence (day 0). Differentiation was carried out approximately according to Iwata et al (Diabetes 2001 50: 1083-1092). Briefly, differentiation was induced by changing to growth medium containing 3-isobutyl-1-methylxanthine (0.5mM), dexamethasone (0.25µM) and 1µM insulin for 3 days, and then growth medium containing 0.8µM insulin for another 3 days. Finally cells were retained in serum free growth medium until ready for experimentation, typically cells were used between days 6-9.

### Preparation of samples.

Pine nut oil (PNO) and palm kernel oil (PKO) fatty acids were prepared by saponification of pine nut oil or palm kernel oil. PNO contains high levels of PUFAs, in particular high levels of pinolenic acid. PKO has high levels of medium chain fatty acids (C8-12). All samples were prepared as 1mg/ml stocks in ethanol and diluted in media accordingly with the following exceptions. IGF-1 was prepared by initially dissolving in 100µl 10mM HCl, followed by the addition of 400µl PBS supplemented with 1mg/ml BSA. This was diluted with media to the correct concentration. Ascorbic acid, sodium vanadate and aspartame were made up in DMEM.

### Evaluation of glucose uptake

Differentiated adipocytes were incubated for a period of 3 days in DMEM (containing 2mM L-glutamine, 100iu/ml penicillin, 100µg/ml streptomycin, 1nM insulin and 10% FCS) supplemented with or without CLA and/or compound As. The cells were then washed three times with DMEM (containing 1mM deoxyglucose). Insulin stimulated glucose uptake was carried out according to Iwata et al (Diabetes 2001 50: 1083-1092). Briefly, cells were stimulated with 1nM insulin in Krebs Ringer Phosphate (KRP) and HEPES buffer [10mM HEPES, 131.2mM NaCI, 4.7mM KCI, 2.5mM CaCl₂, 1.2mM MgSO₄, 2.5mM sodium phosphate buffer, pH 7.4] with 1% BSA at 37°C. After 1-30 minutes the assay was initiated by addition of 10µl KRP containing 2.5µCi [³H]2-deoxyglucose and 1mM deoxyglucose. After 1hr the reaction was terminated by washing the cells 3x with ice-cold DMEM. Cells were then solubilised with 500µl/well of 1% Triton X-100, 0.2% SDS and 0.2N NaOH for 20 minutes at 37°C. Radioactive incorporation was measured by scintillation counting of 100µl of each cell lysate.

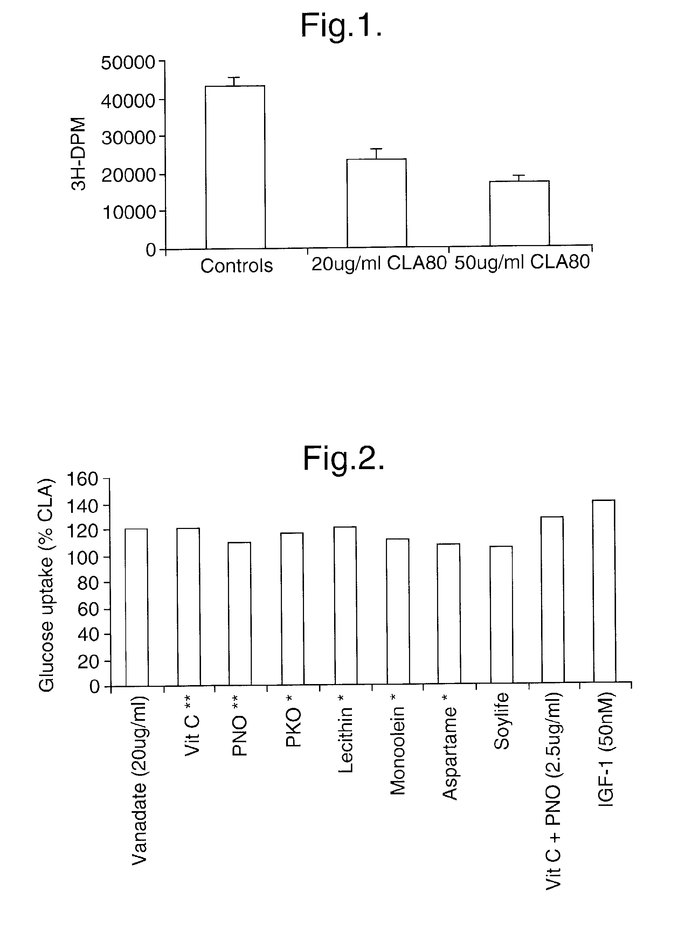

The data illustrating the effects of CLA alone on glucose uptake is represented by the radiolabel incorporation (3H-DPM) during the experiment. The data showing the effects of compound A's in combination with CLA is represented as a percentage of the CLA treatment alone. So any value above 100% demonstrates an increase in glucose uptake over CLA alone.( cf Fig 1 and 2 ).

Typical examples of components A that were found as suitable are components selected from the group consisting of pine nut oil, palm kernel oil, ascorbic acid and salt and ester derivatives thereof (in particular Na and K salts and fatty acid esters, monoglycerides e.g. monoolein, vanadium salts e.g. sodium vanadate, phospholipids e.g. lecithin, sweeteners e.g. aspartame, phytoestrogen containing extracts e.g. Soylife, containing isoflavones.

An additional compound A could be IGF-1, purified or in natural extracts e.g. new zealand deer antler, or compounds or extracts known to boost IGF-1 *in vivo* e.g. DHEA, essential amino acids such as arginine, lysine and ornithine, or colostrum, milk, calcium, cowpea, lupin, zinc supplements.

Additional benefits may also be found from combinations of >1 compound A in combination with CLA.

The mixtures according to the invention preferably comprise the CLA derivative and component A in weight ratios of CLA-derivative (calculated as free CLA ) to component A of 1:99 to 99:1, preferably 80:20 to 20 to 80 most preferably 30 to 70 to 70 to 30.

CLA can comprise about 8 different isomeric isomers (cis/ trans isomers). It was found that from these isomers the trans10 cis9 isomers are the most effective for use in dietic food and therefore we prefer to use as CLA glycerides and / or the CLA fatty acids and / or the CLA alkyl esters those derivatives that are rich in the trans10 cis12-CLA isomer, preferably having more than 30 wt % trans10cis12-CLA, more preferably more than 50 wt % trans10cis12-CLA and in particular more than 70 wt % of the trans10cis12-CLA isomer.

The mixtures as defined above can be used as such but often it is advantageous to use them as blend with another component. Preferred other components are fats and therefore we prefer to apply a blend of one or more vegetable fat (s) and at least 3 wt%, preferably 5 to 35 wt% , most preferably 7 to 25 wt% of the mixtures according to the invention. In order to obtain good mouthfeel we prefer to use a blend , wherein the total fat phase displays a solid fat content measured on a non-stabilised fat by NMR - pulse at the temperature indicated of: 5 to 90 at 5 oC; 2 to 80 at 20 oC and less than 15 at 35 oC. Non stabilised meaning that the fat after being melted at 80 oC was cooled to 0 oC and kept at 0 oC for 30 min, after which the fat is heated to measurement temperature and kept thereon for another 30 min whereupon its solid fat content was measured by NMR -pulse

The mixtures and blends as defined above can be used in CLA rich diet food wherein the diet food comprises a food or drink component, 1 or more component As, capable of stimulating glucose up-take in adipocytes from mammals and wherein component A is present in amounts corresponding with a daily intake of 1mg to 10 grams of component A per day.
It is also possible to use our mixtures or blends in encapsulated food supplements. Hereto encapsulated products are made that comprise an effective amount of the mixture according to the invention in encapsulated form ,whereby the encapsulating material preferably is selected from the group consisting of starch materials, modified starch materials , gelatin , sugars , gums, hydrocolloids

The CLA and component A containing mixtures or blends can also be used in food products. These food products will comprise the mixture or the blend according to the invention.
Typical foods can be selected from the group consisting of spreads (low fat or full fat) , dressings , mayonnaises , cheese , creams, ice creams , ice cream coatings , confectionery coatings , fillings , sauces, culinary products and baked goods, bars, drinks, soups, dairy based drinks, powders and health drinks.

According to another embodiment our invention also comprises the use of a mixture of CLA glycerides, and / or CLA-fatty acids and or CLA-alkyl esters and other components, (= component A) according to the invention for the production of a food or a food supplement with the ability to stimulate glucose up-take in the adipocytes from mammals.

More specifically our invention also concerns the use of a CLA rich diet food wherein the diet food comprises conventional food components , 1 or more component A's and CLA in amounts corresponding with a daily intake of at least 2g CLA per day , preferably at least 5g per day most preferably 6 to 10g per day and wherein the diet food is used to stimulate the glucose up-take by adipocytes from mammals.

More preferably our invention concerns the use of the mixtures or blends wherein the blend comprises one or more vegetable fat (s) and at least 3 wt% , preferably 5 to 35 wt% , most preferably 7 to 25 wt% of the mixtures according to the invention.
The food product produced in particular is a food product selected from the group consisting of spreads (low fat or full fat) , dressings , mayonnaise , cheese , creams , ice creams , ice cream coatings , confectionery coatings , fillings , sauces, culinary products and baked goods

According to a last embodiment our invention also concerns a method of stimulating the glucose up-take by adipocytes from mammals by administering the mammals an effective daily amount of a mixture according to the invention or a blend according to the invention.

Effective amount in this patent application being defined as that amount that gives a noticable effect. This amount might be different for the different compositions and even for the different humans consuming the compositions but this amount can be determined easily by the man skilled in the art.

### Examples

### Example 1: The effects of CLA on Insulin stimulated glucose uptake in 3T3-L1 adipocytes

The insulin stimulated glucose uptake (ISGU) of control differentiated adipocytes were compared with those treated with CLA fatty acids (50:50 ratio of the main isomers, should we include here CLA A807) at a concentration of 10 or 50µg/ml. As shown in Fig. 1, CLA reduces ISGU compared with the control.

### Example 2: Effects of compound A's on ISGU in adipocytes

As shown in Figure 2, a number of compound A's boosts glucose uptake above CLA alone. The increase in glucose uptake in combination with CLA by Vit C (20%), PNO (21%), vit C + PNO (27%) and IGF-1 (39%) in particular were significantly higher than CLA alone. P<0.05 as determined using a Students T-test.

## Claims

**1.** A mixture of CLA glycerides, and / or CLA-fatty acids and or CLA-alkyl esters and another 1 or more components, wherein the other component (= component A) is selected for its capacity to stimulate glucose up-take in adipocytes from mammals , using an appropriate in vitro glucose up-take assay wherein the glucose up-take stimulation is at least 4 %, preferably at least 20% better by use of the blend of the CLA derivative and component A when compared to the use of CLA or its derivative only.

**2.** Mixture according to claim 1 wherein component A is selected from at least one member of the group consisting of pine nut oil acids or esters, pinolenic acid or esters, palm kernel acids or esters, ascorbic acid and the salt and ester derivatives thereof, vanadium salts, lecithin, monoglycerides, aspartame, soy extracts, IGF-1 or IGF-1 boosting agents.

**3.** Mixture according to claims 1 or 2 wherein the weight ratio of CLA-derivative (calculated as free CLA ) to component A is 1:99 to 99:1, preferably 80:20 to 20 to 80 most preferably 30 to 70 to 70 to 30.

**4.** Mixture according to claims 1 to 3 wherein the CLA glycerides and / or the CLA fatty acids and / or the CLA alkyl esters are rich in the trans10cis12-CLA isomer, preferably having more than 30 wt % trans10cis12-CLA, more preferably more than 50 wt % trans10cis12-CLA and in particular more than 70 wt % of the trans10cis12-CLA isomer.

**5.** A blend of one or more vegetable fat (s) and at least 3 wt%, preferably 5 to 35 wt%, most preferably 7 to 25 wt% of the mixtures according to claims 1 to 4.

**6.** Blend according to claim 5 wherein the total fat phase displays a solid fat content measured on a non-stabilised fat by NMR - pulse at the temperature indicated of: 5 to 90 at 5 oC; 2 to 80 at 20 oC and less than 15 at 35 oC

**7.** A CLA rich diet food wherein the diet food comprises a food component, a component A, capable of stimulating glucose up-take in adipocytes from mammmals and wherein component A is present in amounts corresponding with a daily intake of 1 mg to 10 grams of component A per day.

**8.** Food supplement comprising an effective amount of the mixture according to claims 1 to 4 in encapsulated form, whereby the encapsulating material preferably is selected from the group consisting of starch materials , modified starch materials , gelatine , sugars, gum, hydrocolloids.

**9.** Food products comprising the mixture or the blend according to claims 1 to 4 resp 5 to 6.

**10.** Food products according to claim 9, wherein the food product is selected from the group consisting of spreads (low fat or full fat) , dressings , mayonnaise , cheese , creams, ice creams , ice cream coatings , confectionery coatings , fillings , sauces, culinary products, baked goods, bars, drinks, soups, dairy based drinks, powders and health drinks.

**11.** Use of a mixture of CLA glycerides, and / or CLA-fatty acids and or CLA-alkyl esters and another component, (= component A) according to claims 1 to 4 for the production of a food or a food supplement with the ability to stimulate glucose up-take in the adipocytes from mammals

**12.** Use of a CLA rich diet food wherein the diet food comprises conventional food components and a component A, wherein the diet food according to claim 7 is used to stimulate the glucose up-take in adipocytes from mammals.

**13.** Use according to claims 11 to 12 wherein the blend comprises one or more vegetable fat (s) and at least 3 wt% , preferably 5 to 35 wt% , most preferably 7 to 25 wt% of the mixtures defined in claims 1 to 4.

**16.** Use according to claim 11 wherein the food product produced is a food product selected from the group consisting of spreads (low fat or full fat), dressings , mayonnaises , cheese , creams , ice creams , ice cream coatings , confectionery coatings , fillings , sauces, culinary products and baked goods, bars, drinks, soups, dairy based drinks, powders and health drinks.

**15.** Method for stimulating the glucose up-take in adipocytes from mammals by administering the mammals an effective daily amount of a mixture according to claims 1 to 4 or a blend according to claims 5 to 6
